# EUROPEAN PATENT APPLICATION

(11) **EP 4 212 187 A1**
(43) Date of publication of application: **19.07.2023**
(21) Application number: 23151099.1
(22) Date of filing: 11.01.2023
(51) Int. Cl.: A61L 2/20, B67C 7/00

(54) **VESSEL STERILIZER**

(30) Priority: 13.01.2022 JP 2022003888
(71) Applicant: Shibuya Corporation, Kanazawa-shi, Ishikawa 920-8681 (JP)
(72) Inventor: Washi, Naoya, Kanazawa-shi, Ishikawa, 920-8681 (JP)
(74) Representative: Hoffmann Eitle

(57) **Abstract**

A vessel sterilizer includes an endless conveyor provided with a gripper that holds a vessel having an opening facing upward; a nozzle that is provided above a conveyance route of the vessel, wherefrom sterilizing gas is injected from the bottom end of the nozzle toward the opening of the vessel to supply the sterilizing gas inside the vessel; and a swing mechanism that horizontally swings the gripper with respect to the endless conveyor. The swing mechanism swings the gripper forward in the endless conveyor running direction when the vessel held by the gripper approaches the underside of the nozzle and swings the gripper rearward in the endless conveyor running direction when the vessel held by the gripper moves away from the nozzle, thereby the opening of the vessel held by the gripper is maintained below the nozzle for a relatively longer period of time.

## Description

### BACKGROUND OF THE INVENTION

### 1. Field of the Invention

The present invention relates to a vessel sterilizer that sterilizes a moving vessel with sterilization gas injected from a fixed nozzle.

### 2. Description of the Related Art

There is known a sterilizer that sterilizes the inside of a resin vessel via the vessel mouth by injecting sterilizing gas from a sterilizing nozzle provided on a conveyor path of the vessel while holding the resin vessel with a gripper provided on a rotating wheel, see Japanese Patent No. 4978706 and Japanese Patent No. 5892206. For example, in Japanese Patent No. 4978706 and Japanese Patent No. 5892206, a mist of hydrogen peroxide solution (sterilizing gas) is injected from a fixed nozzle arranged above a vessel being transported, and the sterilizing gas is supplied into the vessel while the mouth of the vessel passes directly below the nozzle.

### SUMMARY OF THE INVENTION

However, when the rotation speed of the rotating wheel (vessel conveying speed) is increased to increase the process capacity, the time for the vessel to pass below the nozzle is shortened. Thereby, the amount of sterilizing gas supplied to the inside of the vessel is reduced and it would result in insufficient sterilization. Therefore, a conventional system would need an arrangement of a large number of nozzles for sterilization.

One aspect of the present invention is to increase the amount of sterilizing gas supplied into a vessel from one nozzle even when the vessel is conveyed at high speed.

According to one aspect of the present invention, a vessel sterilizer includes an endless conveyor provided with a gripper that holds a vessel having an opening facing upward; a nozzle that is provided above the conveyance route of the vessel that is conveyed by the endless conveyor, the nozzle injects sterilizing gas from a spout formed at the bottom end of the nozzle and the sterilizing gas from the nozzle is injected toward the opening of the container to supply the sterilizing gas inside the vessel; and a swing mechanism that horizontally swings the gripper with respect to the endless conveyor. The swing mechanism swings the gripper forward in the running direction of the endless conveyor when the vessel held by the gripper approaches below the nozzle and swings the gripper rearward in the running direction of the endless conveyor when the vessel held by the gripper moves away from the nozzle, thereby the opening of the vessel held by the gripper is maintained below the nozzle for a relatively longer period of time.

### BRIEF DESCRIPTION OF THE DRAWINGS

The objects and advantages of the present invention will be better understood from the following description with references to the accompanying drawings in which:
FIG. 1 is a plan view illustrating an arrangement of part of a vessel sterilizer of an embodiment of the present invention;
FIG. 2 is a partially enlarged vertical cross-sectional views showing the configuration of the outer peripheral portion of the rotating wheel that holds the vessels in the external sterilization section;
FIG. 3 is a partially enlarged vertical cross-sectional view showing the configuration of the outer peripheral portion of the rotating wheel that holds the vessels in the internal sterilization sections;
FIG 4 is a perspective view illustrating the configuration of the gripper;
FIG 5 is a side sectional view of the sterilizing gas injection wheel showing the configuration of the gripper in the first and second internal sterilization sections;
FIG 6 is plan view schematically illustrating the operation of the gripper in the first and second internal sterilization sections; and
FIG7 is a plan view schematically illustrating the operation of the gripper in the first and second internal sterilization sections.

### DESCRIPTION OF THE PREFERRED EMBODIMENTS

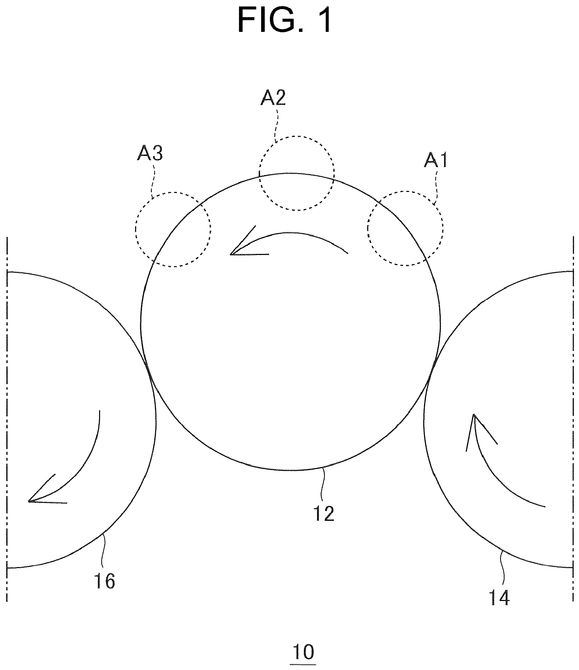

The present invention is described below with references to the embodiments shown in the drawings. Fig. 1 is a plan view illustrating an arrangement of part of a vessel sterilizer of an embodiment of the present invention.

The vessel sterilizer of the present embodiment includes a sterilizing-gas injecting wheel (an endless conveying means) 12 and a rotary-type hot water rinser, which is not shown. Empty vessels V are supplied to the sterilizing-gas injecting wheel 12 from an inlet wheel 14 and sterilizing gas, such as hydrogen peroxide gas, is sprayed on both the outside and inside of the vessels V. A vessel V subjected to sprayed sterilizing gas is transported to a dry air injecting wheel and the rotary-type hot water rinser on the down stream side via an outlet wheel 16.

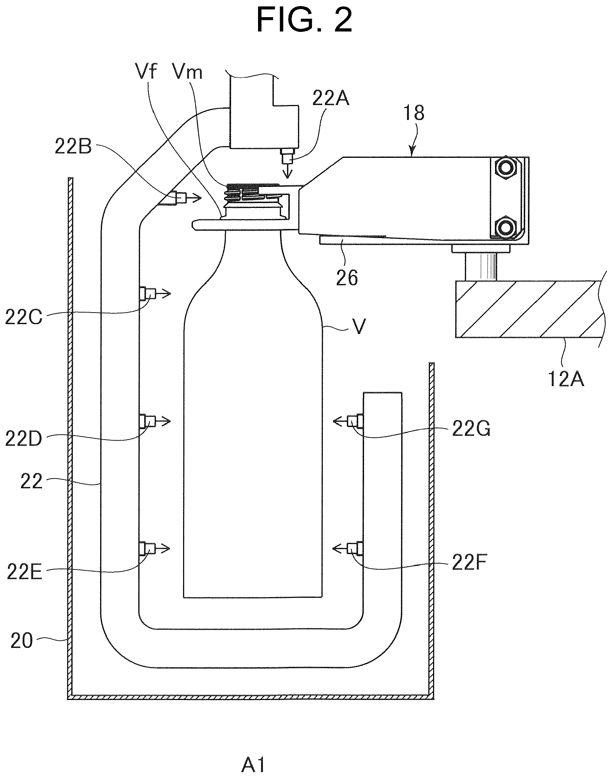

In this embodiment, the vessel V may be a resin bottle configured with a screw thread on the outer periphery of the mouth portion Vm and a flange Vf protruding outward below the mouth portion Vm (see FIG. 2). In neighboring wheels, the vessels V are alternately gripped by grippers, above and below the flanges Vf and conveyed continuously. In the sterilizing-gas injecting wheel 12, the vessels V are conveyed along the outer circumference of the rotating wheel 12A, and on this route is provided an external sterilization section A1 and first and second internal sterilization sections A2 and A3 from the upstream side. While the vessel V is being conveyed through each section, sterilizing gas is injected or sprayed on the opening Vm, on the outside and into the inside of the vessel V. Note that the order of injecting the sterilizing gas to each part of the vessel is not limited to that of this embodiment, and the order of the sections A1, A2, and A3 is arbitrary. In the present embodiment, two internal sterilization sections are provided, but the number of such sections may be one or three or more.

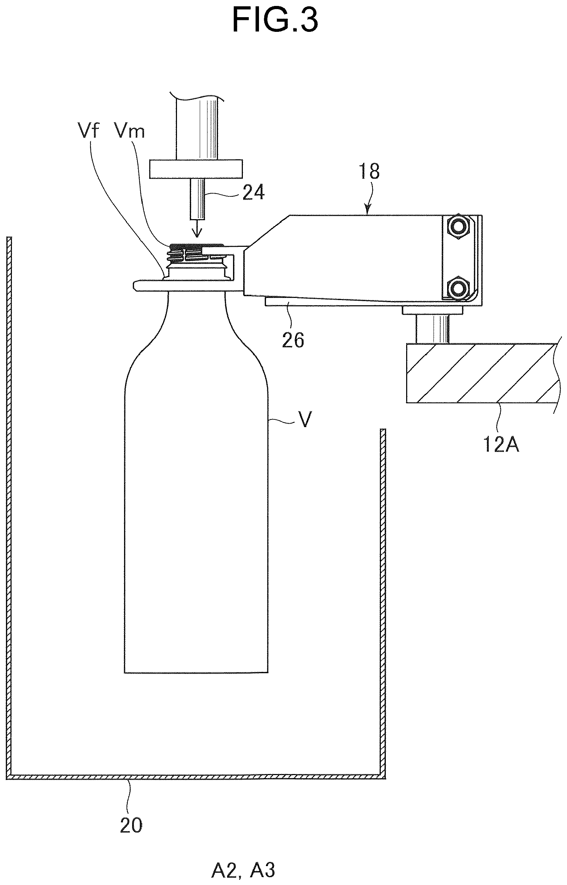

FIGS. 2 and 3 are partially enlarged vertical cross-sectional views showing the configuration of the outer peripheral portion of the rotating wheel 12A that holds the vessels V in the external sterilization section A1 and the internal sterilization sections A2 and A3, respectively.

As shown in FIGS. 2 and 3, the vessels V are held by grippers 18 of the sterilizing gas injecting wheel 12 arranged at predetermined intervals along the outer circumference of the rotating wheel 12A. The vessels V are supported below the flanges Vf so that they are suspended by the grippers 18 and conveyed by the sterilizing gas injecting wheel 12. A hood 20 is arranged throughout the external sterilization section A1 and the first and second internal sterilization sections A2 and A3 so as to cover both sides and the bottom of the conveyance path of each vessel V conveyed by a gripper 18. The hood 20 is arranged in an arc shape along the outer circumference of the rotating wheel 12A. The upper end of the radially inner side wall of the hood 20 extends to a height lower than the gripper 18 to not interfere with the gripper 18. On the other hand, the upper end of the radially outer side wall may extend to a height slightly higher than the mouth portion Vm of the vessel V.

As shown in FIG. 2, in the external sterilization section A1, a fixed sterilizing gas supply pipe 22 is arranged above the opening Vm and along the inner surface of the hood 20 so as to surround the vessel V being transported. The sterilizing gas supply pipe 22 is provided with a plurality (e.g., seven nozzles) of nozzles 22A, 22B, 22C, 22D, 22E, 22F and 22G along the pipe. The nozzle 22A is arranged above the mouth portion Vm of the vessel V held by the gripper 18 and injects the sterilizing gas downward along the outer surface of the mouth portion Vm of the vessel V on the inner side with respect to the wheel radial direction. The nozzles 22B, 22C, 22D, and 22E are arranged so as to inject the sterilizing gas toward the outer surface of the mouth, shoulder, center, and bottom sections of the vessel V, respectively, on the outer side with respect to the wheel radial direction. Furthermore, the nozzles 22F and 22G are arranged so as to inject the sterilizing gas toward the outer surface of the bottom and central portions of the vessel V, respectively, on the inner side with respect to the wheel radial direction.

As illustrated in FIG. 3, in the first and second internal sterilization sections A2 and A3, the fixed nozzles 24 are arranged so as to inject the sterilization gas downward to about the center of the mouth portion Vm and into the interior of the vessel V.

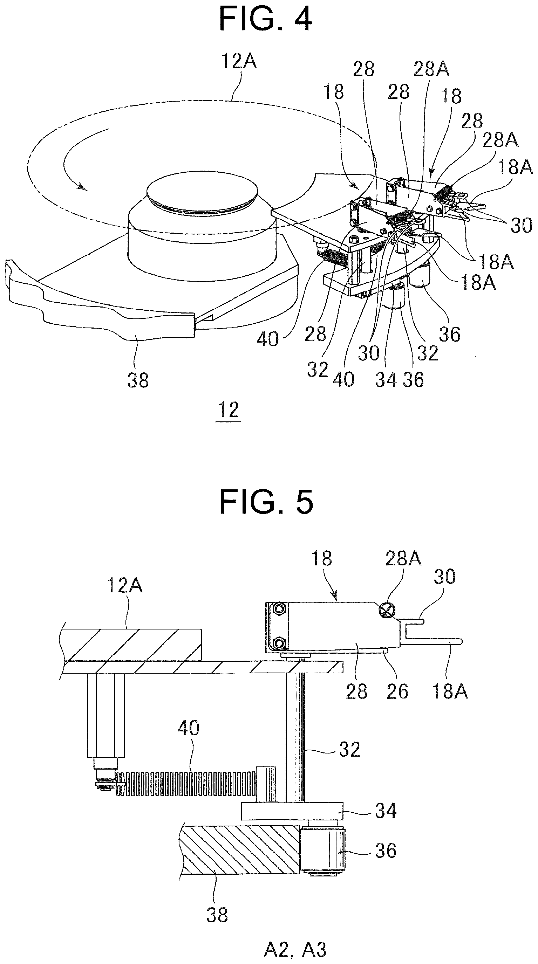

Next, the configuration of the gripper 18 of the present embodiment will be described with reference to FIGS. 4 and 5. FIG. 4 is a perspective view illustrating the configuration of the gripper 18, and FIG. 5 is a side sectional view of the sterilizing gas injection wheel 12 showing the configuration of the gripper 18 in the first and second internal sterilization sections A2 and A3.

The gripper 18 includes a pair of gripping members 18A for gripping the neck of the vessel V below the flange Vf. The base end side of each gripping member 18A is supported by flat springs 28 attached to the right and left sides of a base 26. A biasing member 28A such as a spring is disposed between the flat springs 28, and both ends of the biasing member 28A are attached to each flat spring 28. As a result, the flat spring 28 or the gripping members 18A are biased toward each other.

A side support member 30, which is integrally provided above the base end of each gripping member 18A, makes contact with the side surface of the mouth portion Vm of the vessel V and supports the mouth portion Vm from the side. When the vessel V is pushed between the gripping members 18A, the lower portion of the flange Vf of the vessel V is gripped by the gripping members 18A and the side support member 30 abuts against the side of the mouth portion Vm. As a result, the rotating vessel V is stably held by the gripping members 18A and the side support members 30.

The base end of the base 26 of the gripper 18 is integrally attached to the upper end of a vertically arranged rotating shaft 32, and the distal end of the gripper 18 can horizontally swing around the rotating shaft 32 via a swing mechanism. The rotating shaft 32 is rotatably supported on the outer peripheral portion of the rotating wheel 12A, and the lower end of the rotating shaft 32 is attached with a swing lever 34. One end of the swing lever 34 is provided with a cam follower 36 that engages a fixed cam 38 provided on the sterilizing gas injection wheel 12. The swing lever 34 is also attached to one end of a spring 40, the other end of which is attached to the rotating wheel 12A. Thereby, the swing lever 34 is biased so that the tip of the swing lever 34 provided with the cam follower 36 is urged radially inward with respect to the rotary wheel 12A. Although the cam 38 is partially illustrated in FIG. 4, the cam 38 is actually arranged over the entire circumference of the rotating wheel 12A.

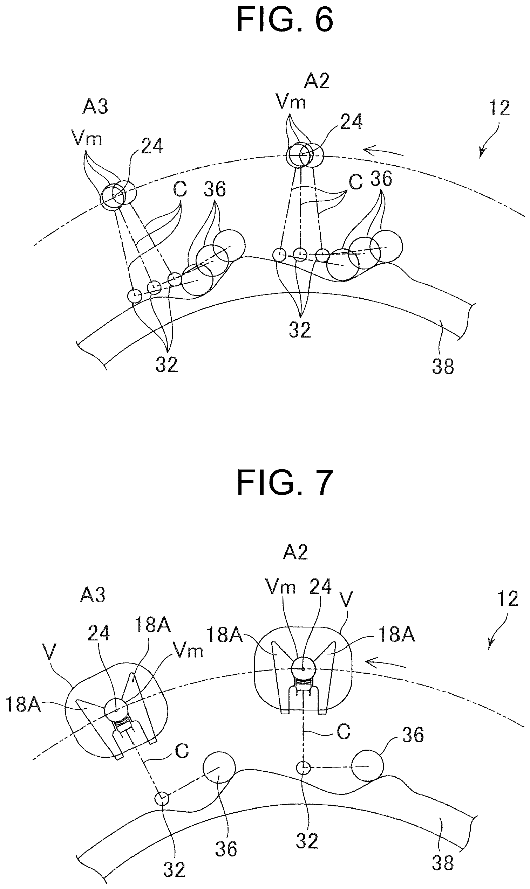

FIGS. 6 and 7 are plan views schematically illustrating the operation of the gripper 18 caused by the engagement between the cam follower 36 and the cam 38 in the first and second internal sterilization sections A2 and A3. FIG. 6 illustrates the positions of the nozzles 24 in the first and second internal sterilizing zones A2 and A3, the positions of the cam followers 36 when grippers 18 pass through the first and second internal sterilizing zones A2 and A3, the positions of the centerlines C of the grippers 18, and the positions of the mouth portions Vm of the vessels V held by the grippers 18, in three positons at different phases. The figure illustrates three situations: (1) When the rotating shaft 32 is located at a position slightly before reaching the position of the nozzle 24 in the circumferential direction, (2) When the rotational axis 32 is positioned at the same circumferential position as the nozzle 24, and (3) When the rotational axis 32 slightly circumferentially passes over the nozzle 24. On the other hand, FIG. 7 illustrates the positions of the nozzles 24 and the positions of the cam followers 36, the centerlines C of the grippers 18, the grippers 18, the vessels V held by the grippers 18 and their mouths Vm, when the rotary shaft 32 circumferentially reaches the same position as the nozzle 24 and the center line C of the gripper 18 is positioned in the radial direction of the rotary wheel 12A. Incidentally, the nozzle 24 has a smaller diameter than the mouth portion Vm of the vessel V as illustrated in FIGS. 6 and 7.

Outside of the internal sterilizing zones A2 and A3, the centerline of the gripper 18 is positioned along the radial direction of the rotary wheel 12A by an engagement of the cam follower 36 and the cam 38. As the cam follower 36 enters the first internal sterilization section A2, it is gradually pushed outward in the radial direction of the rotating wheel 12A by the cam 38. Thereby, the gripper 18 is rotated about the rotating shaft 32 such that its centerline advances in the vessel-conveying direction before the rotary shaft 32 reaches the same circumferential position as the nozzle 24 in the first internal sterilization section A2. Namely, the gripper 18 is rotated forward in the conveying direction, and the mouth portion Vm of the vessel V held by the gripper 18 is moved below the nozzle 24 of the first internal sterilization section A2.

The cam follower 36 is then moved radially inward with respect to the rotating wheel 12A along the cam 38 as either the rotating wheel 12A is rotated or the rotating shaft 32 is advanced. Thereby, the gripper 18 is rotated rearward in the conveying direction in accordance with the rotational speed of the rotary wheel 12A. This keeps the mouth Vm of the vessel V held by the gripper 18 below the nozzle 24 of the first internal sterilization section A2.

As the rotary wheel 12A is rotated or the rotating shaft 32 is advanced, the cam follower 36 is moved inward to a position where the centerline C of the gripper 18 is positioned in the radial direction of the rotary wheel 12A and further moved radially inward. As a result, the gripper 18 is rotated rearward in the conveying direction in accordance with the rotational speed of the rotary wheel 12A. Thereby, the mouth portion Vm of the vessel V held by the gripper 18 is continuously maintained below the nozzle 24 of the first internal sterilization section A2.

Thereafter, as the rotary wheel 12A is further rotated or the rotating shaft 32 is further advanced, the cam follower 36 is again pushed radially outward beyond the position where the centerline C of the gripper 18 is positioned in the radial direction. Thereby, the mouth portion Vm of the vessel V held by the gripper 18 is moved toward the position below the nozzle 24 of the second internal sterilization section A3. In the second internal sterilization section A3, the cam follower 36 moves along the cam 38 as in the first internal sterilization section A2. That is, the cam follower 36 is pushed radially outward again. When the centerline C of the gripper 18 is positioned in the radial direction of the rotating wheel 12A, the cam follower 36 moves along the cam 38 to maintain-as much as possible-the mouth portion Vm below the nozzle 24.

As described above, according to the vessel sterilizing apparatus of the present embodiment, the gripper is swung forward in the rotation direction of the rotating wheel so that the mouth (opening) is pushed out below a nozzle when the vessel held by the gripper approaches the nozzle. And when the mouth moves away from the nozzle, the gripper is swung rearward in the direction of rotation of the rotating wheel so that the mouth of the conveyed vessel can be maintained below the nozzle for a longer period of time (for a predetermined time). Thereby, the amount of sterilizing gas supplied into the vessel from one nozzle is increased even when the vessel is conveyed at high speed.

The vessel sterilizer of the present embodiment sprays the sterilizing gas on the vessel conveyed in the circumferential direction since the vessel is conveyed by the gripper provided on the outer circumference of the rotating wheel. However, instead of the sterilizing gas injection wheel of the present embodiment, the vessel may be conveyed and sterilized by an endless conveying system having a gripper provided on the outer periphery of an endless chain that is entrained between a pair of sprockets. In this case, the vessel is conveyed by a gripper that alternately transports between a straight section and a curved section, and the sterilizing gas is sprayed into the vessel from a fixed nozzle in either or both of the straight and curved sections. In front of and behind the fixed nozzle, the gripper is moved forward then backward in the conveying direction, respectively, and the mouth of the vessel is maintained for a relatively longer time below one fixed nozzle, as in the present embodiment.

Although the embodiments of the present invention have been described herein with reference to the accompanying drawings, obviously many modifications and changes may be made by those skilled in this art without departing from the scope of the invention.

## Claims

1. A vessel sterilizer, comprising:
an endless conveyor provided with a gripper that holds a vessel having an opening facing upward;
a nozzle that is provided above a conveyance route of the vessel that is conveyed by the endless conveyor, the nozzle injects sterilizing gas from a spout formed at a bottom end of the nozzle and the sterilizing gas from the nozzle is injected toward the opening of the vessel to supply the sterilizing gas inside the vessel; and
a swing mechanism that horizontally swings the gripper with respect to the endless conveyor;
the swing mechanism swings the gripper forward in a running direction of the endless conveyor when the vessel held by the gripper approaches the underside of the nozzle and swings the gripper rearward in the running direction of the endless conveyor when the vessel held by the gripper moves away from the nozzle, thereby the opening of the vessel held by the gripper is maintained below the nozzle for a relatively longer period of time.
